# EUROPEAN PATENT APPLICATION

(11) **EP 1 327 687 A1**
(43) Date of publication of application: **16.07.2003**
(21) Application number: 01961223.3
(22) Date of filing: 30.08.2001
(51) Int. Cl.: C12N 15/86, C12N 15/51, C12N 5/16, C12Q 1/68, C12Q 1/02, G01N 33/15, G01N 33/50, A01K 67/00

(54) **METHODS OF INFECTION WITH HEPATITIS C VIRUS**

(30) Priority: 03.10.2000 JP 2000303374
(71) Applicant: Tokyo Metropolitan Organization for Medical Research, Shinjuku-ku, Tokyo 163-8001 (JP); CHUGAI SEIYAKU KABUSHIKI KAISHA, Tokyo, 115-8543 (JP)
(72) Inventor: KOHARA, Michinori, Kita-ku, Tokyo 114-0014 (JP); KATSUME, Asao, Gotenba-shi, Shizuoka 412-8513 (JP)
(74) Representative: Maschio, Antonio
(86) International application number: JP0107498
(87) International publication number: WO02028174

(57) **Abstract**

The present invention relates to a method for efficiently infecting a small animal with hepatitis C virus. Specifically, the present invention relates to a method for infecting a small animal with hepatitis C virus, which comprises a step of administering RNA containing the genomic RNA of hepatitis C virus into the liver, or a step of administering to a small animal the culture supernatant of an animal cell having a vector that contains cDNA corresponding to the genomic RNA of hepatitis C virus introduced therein, and relates to a small animal infected with hepatitis C virus by these methods. The present invention further relates to a method for screening for remedies against hepatitis C virus-associated diseases, or substances that inhibit the growth of hepatitis C virus using the yield of the virus as an indicator.

## Description

### TECHNICAL FIELD

The present invention relates to methods for efficiently infecting small animals with hepatitis C virus (hereinafter, referred to as "HCV"). The infection methods of the present invention can improve HCV infection rate, so that they are useful in the production of hepatitis C model animals. The present invention further relates to novel screening methods that are useful in searching for remedies against HCV-associated diseases or substances that inhibit the growth of HCV.

### BACKGROUND ART

HCV is a major causative virus of post-transfusion non-A, non-B hepatitis (Saito, I. et al., Proc. Natl. Acad. Sci. USA, 87, 6547-6549 (1990)). Hepatitis caused by this virus develops at a high rate into chronic hepatitis, and cirrhosis and hepatoma, so that hepatitis is a disease for which the discovery of reliable therapeutic measures is an urgent concern. The cDNA of this virus was cloned by Choo et al in 1989 (Choo, Q. -L., et al., Science, 244, 359-362 (1989)), and the virus is known to be a single stranded RNA virus belonging to the Flavivirus family (Kato, N., et al., Proc. Natl. Acad. Sci., USA, 87, 9524-9528 (1990)). To date, the entire nucleotide sequence and the amino acid sequence have been elucidated by several research groups (Kato, N., et al., Proc. Natl. Acad. Sci., USA, 87, 9524-9528 (1990), Proc. Natl. Acad. Sci., USA, 88, 2451- 2455 (1991), J. Virol., 65, 1105-1113 (1991), J. Gen. Virol., 72, 2697-2704 (1991), Virology, 188, 331-341(1992)).

There have been many reports concerning HCV as described above, however, HCV is still poorly understood. For example, the mechanisms of HCV including infection, replication, extracellular release and the like are almost unknown under the present state of knowledge. Elucidation of these mechanisms are delayed because, for example, the number of types of animals that can be infected with HCV is limited, the production of appropriate disease model animals is difficult, and a simple screening system for studying anti-HCV action has not yet been established.

The chimpanzee is known as a non-human animal that can be infected with HCV (Alter, H. J. et al., (1978) Lancet 1, 459-463, Bradley, D.W. et al., (1979) J. Med. Virol. 3, 253-269). However, there are problems such that chimpanzees are few in number, expensive, and difficult to keep, so that they are inappropriate as model animals.

Xie et al reported that tupaias, small Southeast Asian mammals, were infected with HCV (Zhi-Chun Xie et al., Viology 244, 513-520 (1998)). Tupaias are far easier to keep than chimpanzees, and thus the use of tupaias as a new model animal is expected. According to Xie et al's report, however, the infection rate of tupaias with HCV was approximately 30%, not necessarily considered high.

### DISCLOSURE OF THE INVENTION

An object of the present invention is to provide a means for producing more efficiently an HCV model animal by improving the HCV infection rates of various small animals including tupaias. Further, another object of the present invention is to provide a simple screening method for studying anti-HCV action.

As a result of intensive studies to solve the above problems, we have found that the HCV infection rate among small animals is greatly improved by administering HCV genomic RNA (hereinafter, it may also be simply referred to as "HCV RNA") to the liver of the small animal, and by introducing a vector that contains cDNA (hereinafter, it may also be simply referred to as "HCV cDNA") corresponding to the HCV genomic RNA into an animal cell, and then administering the culture supernatant, resulting from culturing of the cell, into the small animal. We have completed the present invention by further finding that a simple screening system using the yield of the virus as an indicator can be established by the use of the culture supernatant of the above HCV cDNA-introduced cells or the above HCV-infected small animals.

That is, the present invention is a method for infecting small animals with HCV, which comprises the following steps of (hereinafter, the method is referred to as "first infection method"):
1) preparing RNA containing HCV genomic RNA
2) administering the above RNA into the liver of a small animal

Further, the present invention is a method for infecting small animals with HCV, which comprises the following steps of (hereinafter, the method is referred to as "second infection method"):
1) introducing a vector that contains cDNA corresponding to the HCV genomic RNA into an animal cell
2) culturing the above cells
3) administering the culture supernatant of the above cultured cells into small animals

Further, the present invention is a small animal infected with HCV by the above method.

Further, the present invention is a screening method for remedies against HCV-associated diseases or substances that inhibit the growth of HCV using the yield of the virus as an indicator.
The above screening method may be:
1) a method which comprises a step of administering a test substance to the above HCV-infected small animal,
2) a method which comprises a step of bringing a test substance in contact with an animal cell that can produce HCV, or
3) a method which comprises a step of bringing a test substance in contact with an animal cell having a vector that contains cDNA corresponding to the HCV genomic RNA introduced therein.

### BRIEF DESCRIPTION OF DRAWINGS

Figure 1 shows the structure of pCALN/HCV RBZ.
Figure 2 shows changes over time in serum HCV RNA levels when HCV RNA was administered to the liver of a tupaia (Individual No. 129).
Figure 3 shows changes over time in serum HCV RNA levels when HCV RNA was administered to the liver of a tupaia (Individual No. 131).
Figure 4 shows changes over time in serum HCV RNA levels when the supernatant of cells allowed to express vectors containing HCV cDNA was administered to a tupaia (Individual No. 135).
Figure 5 shows changes over time in serum HCV RNA levels when the supernatant of cells allowed to express vectors containing HCV cDNA was administered to a tupaia (Individual No. 143).
Figure 6 shows changes over time in serum HCV RNA levels when serum containing HCV RNA was administered (Individual No. 117).
Figure 7 shows changes over time in serum HCV RNA levels when serum containing HCV RNA was administered (Individual No. 165).
Figure 8 shows changes over time in HCV RNA levels after Tam (●: 100 nM Tam added, ○: control (no Tam added)) was added to the culture supernatant of HCV cDNA-introduced cells (IMY42-5).
Figure 9 shows HCV RNA levels after Tam and various concentrations of Cyclosporin A were added to the culture supernatant of HCV cDNA-introduced cells (IMY42-5).
Figure 10 shows HCV RNA levels after Tam and various concentrations of ribavirin were added to the culture supernatant of HCV cDNA-introduced cells (IMY42-5).

This specification includes part or all of the contents as disclosed in the specification and/or drawings of Japanese Patent Application No. 2000-303374, which is a priority document of the present application.

### BEST MODE FOR CARRYING OUT THE INVENTION

The present invention is described in detail as follows.

The first infection method comprises the steps of preparing RNA containing HCV RNA and administering this RNA into the liver of a small animal.

Any small animal that can be infected with HCV by the infection method of the present invention may be used. Preferred examples of such a small animal can include small animals of the order Rodentia, the order Macroscelidea, the order Lagomorpha and the order Scandentia. A particularly preferred small animal is *Tupaia belangeri*.

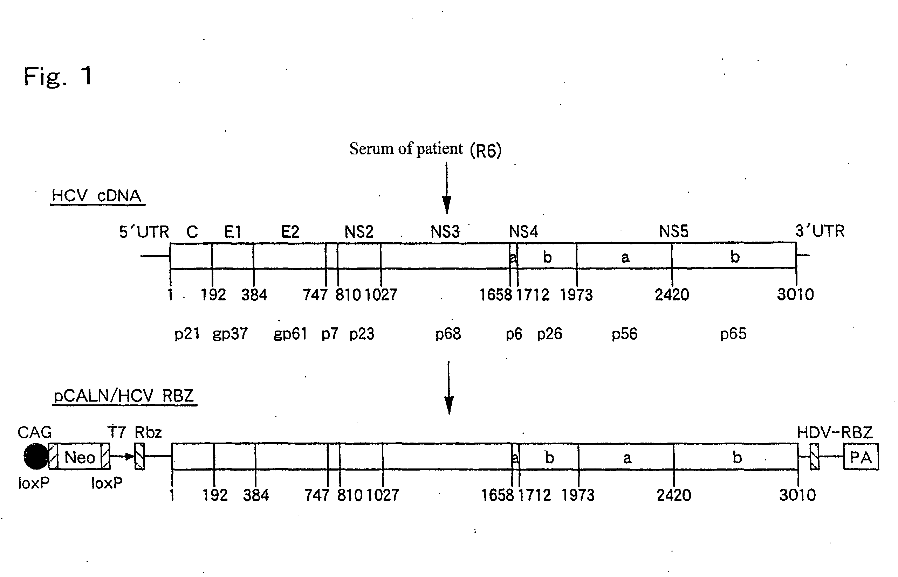

RNA to be prepared herein may be RNA containing a part of HCV RNA, and preferably contains the full length HCV RNA. The full length HCV RNA can be prepared using, for example, pCALN/HCV RBZ. As shown in Fig. 1, pCALN/HCV RBZ contains cDNA corresponding to the full length HCV RNA and sequences corresponding to ribozymes on its both ends. The transcription product of pCALN/HCV RBZ will be the full length HCV RNA by the self processing of the two ribozymes.

RNA may be prepared intracellularly or extracellularly. Extracellular preparation can be performed using, for example, a vector containing HCV cDNA and a commercially available in vitro transcription kit. When transcription reaction of pCALN/HCV RBZ is performed extracellularly, RNA that contains the full length RNA of HCV having extra added sequences may be obtained because of incomplete self processing. Such RNA is also included in the above "RNA containing HCV RNA."

RNA can be administered into the liver by employing a general method for introducing polynucleotides into eukaryotic cells. The liposome method, lipofection method or the like can be exemplified as such a method. The dose is not specifically limited, and approximately 1 to 1000 µg of RNA is preferably administered.

The second infection method comprises the steps of introducing a vector containing HCV cDNA into animal cells, culturing these cells, and administering the culture supernatant of the above cells to small animals.

The cDNA contained in the vector may correspond to a part of the HCV RNA. A preferred cDNA corresponds to the full length HCV RNA. As a vector containing cDNA corresponding to the full length HCV RNA, pCALN/HCV RBZ can be exemplified.

Any animal cell that allows replication of HCV viral particles from an introduced vector may be used. Examples of such animal cells that can be used herein include IMY cells, HuH-7 cells, HepG2 cells, MOLT-4 cells, MT-2 cells, Daudi cells, primary liver cells, and other cells or cell lines derived from liver and hemocyte.

Vectors containing cDNAs can be introduced into animal cells by employing a general method for introducing polynucleotides into eukaryotic cells. As such a method, the liposome method, calcium phosphate method and electroporation method can be exemplified. The amount of vectors to be introduced is not specifically limited, and approximately 1 ng to 1 mg of the vector is preferably introduced.

To increase the yield of HCV viral particles, a vector expressing RNA polymerase may be introduced into an animal cell. For example, in pCALN/HCV RBZ, T7 promoter is located upstream of HCV cDNA (Fig. 1). Thus, introduction of vectors expressing T7 RNA polymerase can increase the yield of HCV viral particles.

A culturing method for animal cells can be determined according to animal cells to be used herein. For example, when IMY cells are used, the cells are preferably cultured in D-MEM media at 37°C for 12 to 72 hours.

The dose of the culture supernatant is not specifically limited. For example, approximately 10 to 10¹⁰ copies are preferably administered. The culture supernatant may be administered intact, or administered after treatment with DNaseI and/or RNase. The route of administration is also not specifically limited. For example, intravenous administration may be performed.

The screening method of the present invention is a screening method for remedies against HCV-associated diseases, or substances that inhibit the growth of HCV using the yield of the virus as an indicator.

The first screening method comprises the steps of administering a test substance to the small animal of the present invention, measuring HCV levels in the small animal, and selecting substances that cause decreases in HCV levels when compared to non-administration of the test substance.

Any small animal that can be infected with HCV by the infection method of the present invention may be used. Preferred examples of such a small animal can include small animals of the order Rodentia, the order Macroscelidea, the order Lagomorpha and the order Scandentia. A particularly preferred small animal is *Tupaia belangeri*.

Further, the second screening method comprises the steps of bringing animal cells that can produce HCV in contact with a test substance, culturing these cells, measuring HCV levels in the culture supernatant of the cells, and selecting substances that cause decreases in HCV levels when compared to non-contact with the test substance.

A preferred example of an animal cell that can produce the above HCV is an animal cell into which, according to the infection method of the present invention, a vector containing cDNA corresponding to HCV genomic RNA has been introduced. Hence, cDNA contained in the vector may be a cDNA corresponding to a part of the HCV RNA, and preferably, is a cDNA corresponding to the full length HCV RNA. As a vector containing cDNA corresponding to the full length HCV RNA, pCALN/HCV RBZ can be exemplified. In addition, animal cells, a method for introducing the vector containing cDNA into an animal cell and a method for culturing animal cells are employed according to the above-mentioned infection method of the present invention.

In the screening method of the present invention, HCV levels of any subject may be measured, as long as the subjects represent directly or indirectly not only HCV itself but also HCV levels. Measuring RNA levels of HCV is simple and preferable. Further, any known measurement method may be used, such as absorbance measurement, RT-PCR, TMA-HPA, PCR-HPA or ELISA.

### [Example 1]

### HCV infection by administering HCV RNA into liver

HCV RNA was synthesized from pCALN/HCV RBZ using a MEGAscript in vitro transcription kit (Ambion). pCALN/HCV RBZ is a vector with which the full length HCV RNA (JP Patent Publication (Unexamined Application) No. 2000-152793) can be synthesized. *Escherichia coli* containing the vector was deposited with the International Patent Organism Depositary, National Institute of Advanced Industrial Science and Technology, Chuo-6, 1-1-1 Higashi, Tsukuba-shi, Ibaraki, Japan (Accession number: FERM BP-6763, Accession date: October 31, 1997).

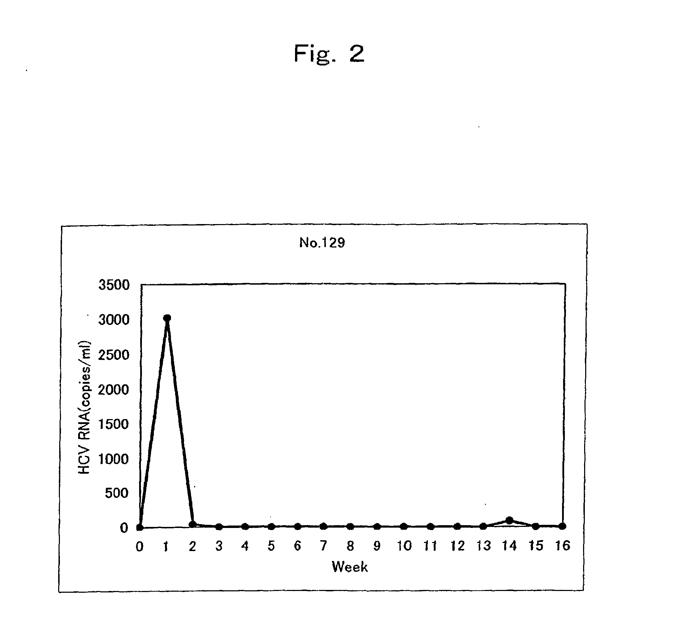

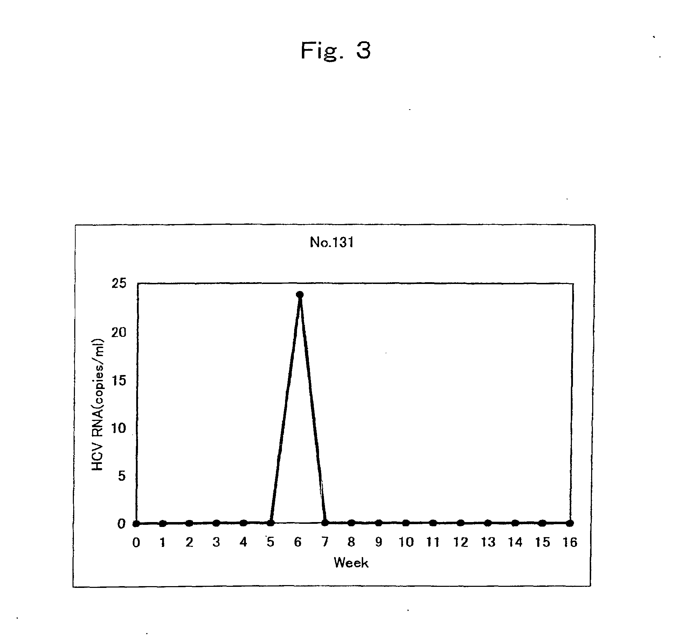

HCV RNA and cationic lipid (DMRIE-C; GIBCO-BRL) were suspended in PBS(-). The suspension was inoculated into the livers of tupaias anesthetized with ketamine hydrochloride (Ketalar; Sankyo) (Individual No. 129 and No. 131). The amounts of inoculated RNA and cationic lipid were 15 µg and 15 µl, respectively. Tupaias inoculated with the RNA were kept under normal conditions, and serum HCV RNA levels were measured over time. Tupaia serum was obtained by collecting and centrifuging blood from the venous plexus of the femur of the tupaia under anesthesia with ketamine hydrochloride. RNA was extracted by the guanidine thiocyanate and phenol/chloroform methods, and then Serum HCV RNA levels were measured by the Real-Time Detection PCR (RTD-PCR) method (T. Takeuchi et al., GASTROENTEROLOGY 116, 636-642 (1999)). The results are shown in Fig. 2 (No. 129) and Fig. 3 (No. 131).

As shown in these figures, increased serum HCV RNA levels were observed for both individuals, so that establishment of infection was confirmed.

### [Example 2]

### HCV infection by administration of culture supernatant of HCV cDNA-introduced cells

15 µg of pCALN/HCV RBZ and 40 µg of cationic lipid (DMRIE-C; GIBCO-BRL) were suspended in Opti-MEM (GIBCO-BRL). The suspension was allowed to stand for 15 minutes at room temperature. 7.8 ml of this suspension was mixed with IMY-N9 cells (Ito, T. et al., Hepatology 34 (2), 2001) cultured in a 10 cm dish. The mixture was incubated at 37°C for 5 hours, so that pCALN/HCV RBZ was transfected into the IMY-N9 cells. Next, adenovirus expressing T7 RNA polymerase (Aoki et al., Virology, 1998, 250:140-150) was added to the IMY cells, and allowed to stand for 1 hour, so that IMY cells were infected with the adenovirus (MOI=20). Since HCV cDNA was located downstream of the T7 promoter, HCV cDNA in pCALN/HCV RBZ was expressed by infection with the adenovirus.

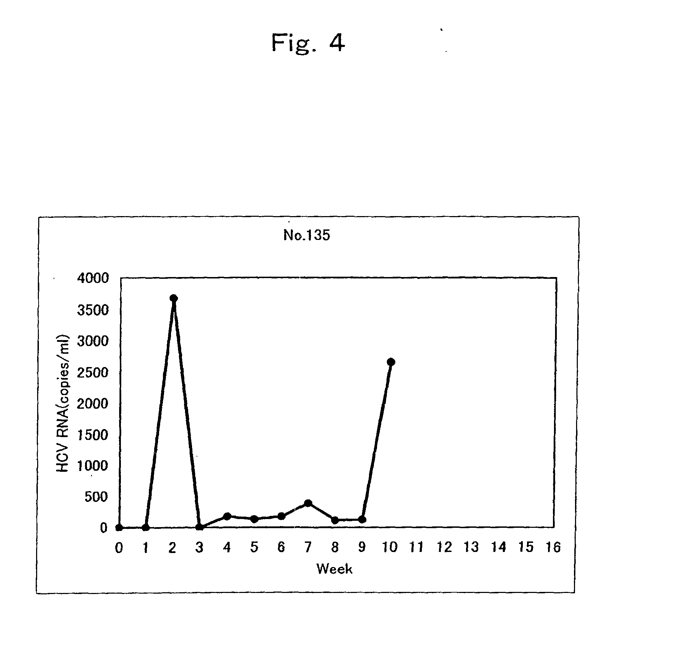

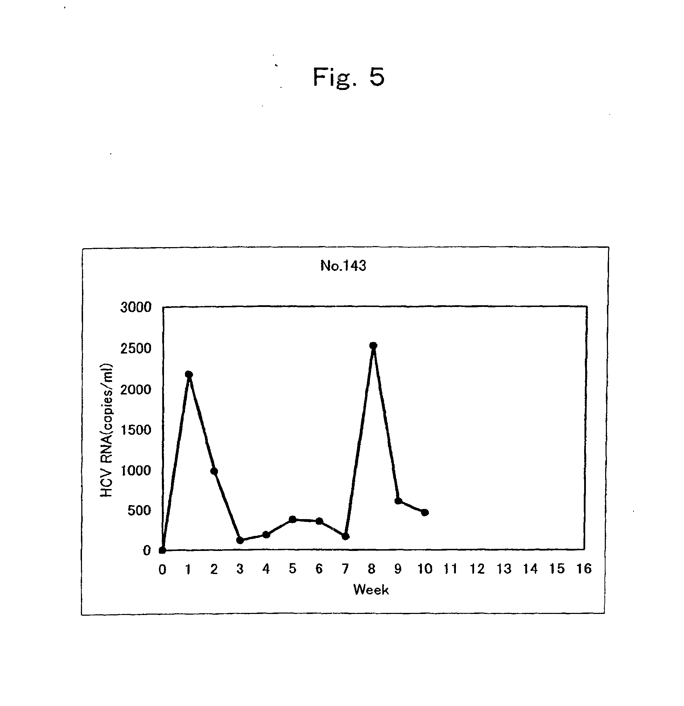

IMY cells, treated as described above, were cultured at 37°C for 12 hours, and then the culture supernatant was collected from the dish. DNaseI (10 unit/0.1ml) was added to the culture supernatant, and reaction was allowed to proceed at 37°C for 10 minutes, and then RNase (5 µg/0.1 ml) was added to the same for the reaction to proceed at 37°C for 10 minutes. After the enzyme treatment, the culture supernatant (containing 10⁷ copies of HCV viral particles) was administered intravenously via the saphenous veins of two tupaias (Individual No. 135 and No. 143). The tupaias administered with the culture supernatant were kept under normal conditions, and serum HCV RNA levels were measured over time in a manner similar to Example 1. The results are shown in Fig. 4 (No. 135) and Fig. 5 (No. 143).

As shown in these figures, increased serum HCV RNA levels were observed for both individuals, so that establishment of infection was confirmed.

### [Comparative example 1]

### HCV infection by administration of serum of HCV patient

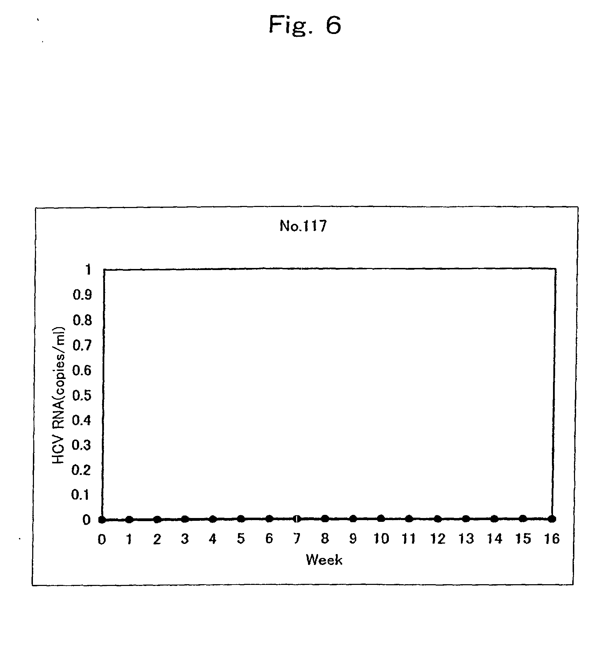

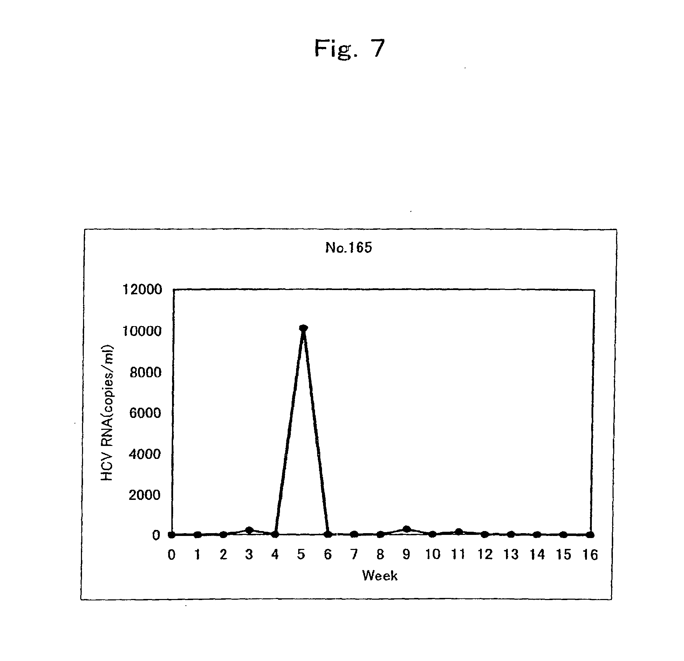

DNase I (10 unit/0.1 ml) was added to the serum collected from HCV patient, for the reaction to proceed at 37°C for 10 minutes. Then RNase (5 µg/0.1 ml) was added to the mixture and reaction was allowed to proceed at 37°C for 10 minutes. After enzyme treatment, the serum (containing 10⁶ copies of HCV viral particles) was administered intravenously via the saphenous veins of two tupaias (Individual Nos: No. 117 and No. 165). RNA-inoculated tupaias were kept under normal conditions, and then serum HCV RNA levels were measured over time in a manner similar to Examples 1 and 2. The results are shown in Fig. 6 (No. 117) and Fig. 7 (No. 165).

As shown in these figures, increased serum HCV RNA levels were observed in one individual (No. 165), while increased serum HCV RNA levels were not observed in the other individual (No. 117). That is, only in one of the two individuals, establishment of HCV infection was confirmed.

### [Example 3]

### Changes over time in the yield of virus in the culture supernatant of HCV cDNA-introduced cells

IMY42-5 cells were prepared by incorporating into IMY-N9 cells, pCALN/HCV RBZ and CAG-Mer-Cre-Mer (Nucleic Acids Res., 1996, 24(4), 543-548) that expresses active Cre recombinant enzyme by adding tamoxifen co-expressed constitutively therein. When tamoxifen acts on the cells, the stuffer region of pCALN/HCV RBZ was looped out and HCV cDNA was expressed.

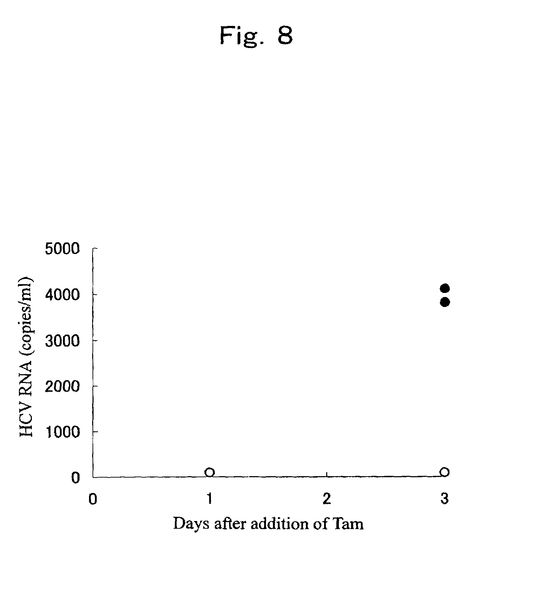

IMY42-5 cells (2.5 x 10⁵/ml) were inoculated, 100 µl/well in a 96-well plate, and then cultured overnight at 37°C under 5% CO₂ atmosphere. Then 4-hydroxi-tamoxifen (Tam) was added at a concentration of 100 nM. On the next day and 3 days after the addition of Tam, the culture supernatant was collected. RNA in the culture supernatant was extracted using ISOGEN-LS (Wako Pure Chemical Industries, Ltd.), and then HCV RNA levels were quantified by the Real-time detection RT-PCR (Takeuchi T. et al; Gastroenterology 1999; 116: 636-642) method. Thus, as shown in Fig. 8, around 4000 copies/ml HCV RNA was detected 3 days after the addition of Tam.

The above results confirmed that viral particles were secreted in the culture supernatant of HCV cDNA-introduced cells, and the experimental system wherein the culture cells were plated was able to easily measure changes in the yield of the virus in many specimens. That is, it was suggested that the method can be effectively used in screening for an anti-viral agent and the like.

### [Example 4]

### Examination of anti-HCV action of drug using the yield of HCV as an indicator (1)

Next, using the method of Example 3, the anti-HCV action of Cyclosporin A (CsA) reported to have anti-HCV action when clinically used in combination with IFN or in the in vitro HCV infection system (Inoue K. et al.; Japanese Journal of Clinical Medicine 2001 Jul; 59(7): 1326-30).

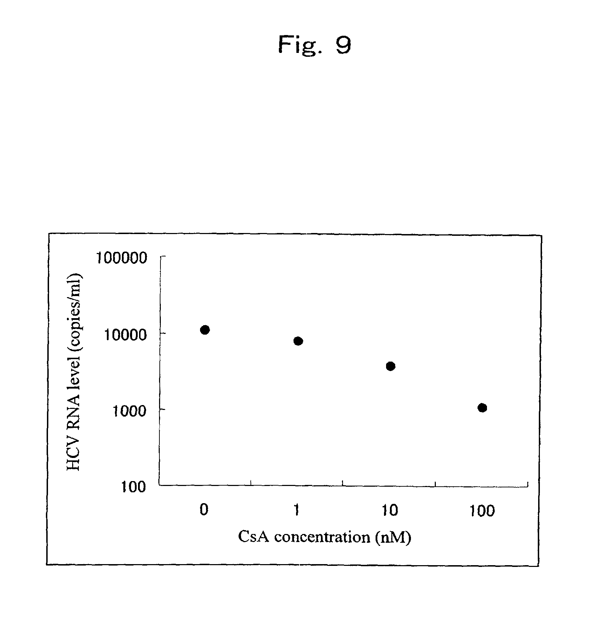

CsA (100, 10, 1 and 0 nM) was added simultaneously with the addition of Tam to IMY42-5 cells. The cells were cultured for 3 days, and then HCV RNA levels in the culture supernatant was measured by Real-time detection RT-PCR. As shown in Fig. 9, concentration-dependent decreases in HCV RNA levels were observed with the addition of CsA.

### [Example 5]

### Examination of anti-HCV action of drug using the yield of HCV as an indicator (2)

The effect of ribavirin, for which a combined use of ribavirin with interferon as an anti-HCV therapeutic agent is currently under clinical trial (ribavirin alone is thought to have no anti-HCV action), was examined.

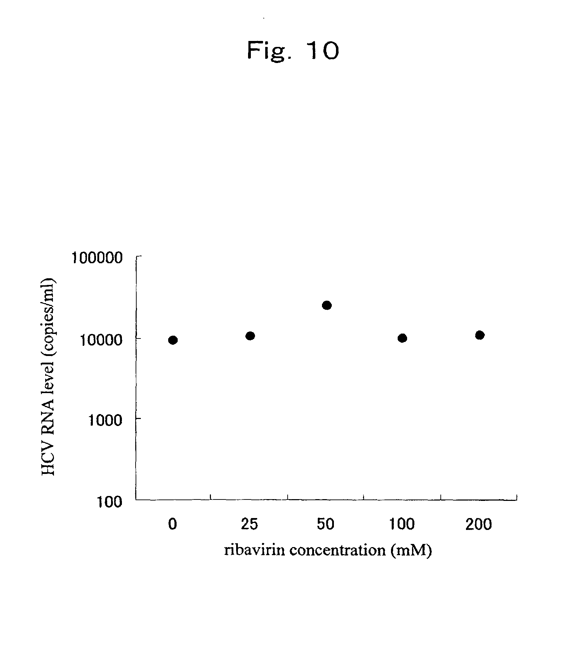

Ribavirin (0, 25, 50, 100, 200 µM) was added simultaneously with the addition of Tam to IMY42-5 cells. The cells were cultured for 2 days, and then HCV RNA levels in the culture supernatant were measured. As shown in Fig. 10, no decrease due to ribavirin was observed in the yield of virus.

As described above, it was confirmed that the screening method of the present invention is useful in screening for remedies against HCV-associated diseases or substances that inhibit the growth of HCV using the yield of the virus as an indicator.

All publications, patents and patent applications cited herein are incorporated herein by reference in their entirety.

### Industrial Applicability

The present invention provides novel methods of infection with HCV. These methods can improve the HCV infection rate of small animals, so that the methods are useful for the preparation of hepatitis C model animals. The present invention further provides novel methods for screening using the yield of the virus as an indicator. These methods are useful in screening for remedies against HCV-associated diseases or substances that inhibit the growth of HCV.

## Claims

1. A method for infecting a small animal with hepatitis C virus, which comprises the following steps of:
1) preparing RNA containing the genomic RNA of hepatitis C virus, and
2) administering said RNA into the liver of a small animal.

2. The method for infecting a small animal with hepatitis C virus of claim 1, wherein the small animal is a tupaia.

3. The method for infecting a small animal with hepatitis C virus of claim 1 or 2, wherein the genomic RNA of a hepatitis C virus is the full length genomic RNA of the hepatitis C virus.

4. The method for infecting a small animal with hepatitis C virus of any one of claims 1 to 3, wherein RNA containing the genomic RNA of hepatitis C virus is prepared using pCALN/HCV RBZ.

5. The method for infecting a small animal with hepatitis C virus of any one of claims 1 to 4, wherein RNA containing the genomic RNA of hepatitis C virus is prepared extracellularly.

6. A small animal, which is infected with hepatitis C virus by the method of any one of claims 1 to 5.

7. A method for infecting a small animal with hepatitis C virus, which comprises the following steps of:
1) introducing a vector that contains cDNA corresponding to the genomic RNA of hepatitis C virus into an animal cell,
2) culturing said cells, and
3) administering the culture supernatant of said cells to a small animal.

8. The method for infecting a small animal with a hepatitis C virus of claim 7, wherein the small animal is a tupaia.

9. The method for infecting a small animal with hepatitis C virus of claim 7 or 8, wherein the genomic RNA of hepatitis C virus is the full length genomic RNA of hepatitis C virus.

10. The method for infecting a small animal with hepatitis C virus of any one of claims 7 to 9, wherein the vector is pCALN/HCV RBZ.

11. A small animal, which is infected with hepatitis C virus by the method of any one of claims 7 to 10.

12. A method for screening for remedies against hepatitis C virus-associated diseases or substances that inhibit the growth of hepatitis C virus, which comprises the following steps of:
1) administering a test substance to the small animal of claim 6 or 11,
2) measuring hepatitis C virus levels in said small animal, and
3) selecting substances that cause decreases in hepatitis C virus levels when compared to non-administration of the test substance.

13. A method for screening for remedies against hepatitis C virus-associated diseases or substances that inhibit the growth of hepatitis C virus, which comprises the following steps of:
1) bringing an animal cell that can produce hepatitis C virus in contact with a test substance,
2) culturing said cells,
3) measuring hepatitis C virus levels in the culture supernatant of said cells, and
4) selecting substances that cause decreases in hepatitis C virus levels when compared to non-contact with the test substance.

14. A method for screening for remedies against hepatitis C virus-associated diseases or substances that inhibit the growth of hepatitis C virus, which comprises the following steps of:
1) bringing an animal cell that has a vector introduced therein containing cDNA corresponding to the genomic RNA of hepatitis C virus in contact with a test substance,
2) culturing said cells,
3) measuring hepatitis C virus levels in the culture supernatant of said cells, and
4) selecting substances that cause decreases in hepatitis C virus levels when compared to non-contact with the test substance.

15. The screening method of claim 14, wherein the vector containing cDNA corresponding to the genomic RNA of hepatitis C virus expresses the full length RNA of HCV.

16. The screening method of claim 14, wherein the vector is pCALN/HCV RBZ.
